(19) Europäisches Patentamt

European Patent Office

Office européen des brevets

(11) **EP 0 897 711 B1**

(12) **EUROPÄISCHE PATENTSCHRIFT**

(45) Veröffentlichungstag und Bekanntmachung des Hinweises auf die Patenterteilung:
**25.10.2000  Patentblatt 2000/43**

(51) Int Cl.7: **A61K 7/06**, A61K 7/08

(21) Anmeldenummer: **98112366.4**

(22) Anmeldetag: **04.07.1998**

(54) **Mittel zur Erhöhung der Formbarkeit und des Glanzes von Haaren**

Product for the improvement of styling and gloss of hair

Produit pour augmenter la façonnage et le brillant des cheveux

(84) Benannte Vertragsstaaten:
**DE ES FR GB IT**

(30) Priorität: **31.07.1997  DE 19733015**
          **16.09.1997  DE 19740651**

(43) Veröffentlichungstag der Anmeldung:
**24.02.1999  Patentblatt 1999/08**

(73) Patentinhaber: **Wella Aktiengesellschaft**
**64274 Darmstadt (DE)**

(72) Erfinder:
 • **Karlen, Thomas, Dr.**
   **3013 Bern (CH)**
 • **Steinbrecht, Karin, Dr.**
   **64372 Ober-Ramstadt (DE)**
 • **Chambettaz, Daniel**
   **1717 St. Ursen (CH)**

(56) Entgegenhaltungen:
**EP-A1- 0 623 336         WO-A1-94/26235**
**US-A- 5 589 157**

**Beschreibung**

Gegenstand der Erfindung ist ein Mittel zur Erhöhung der Formbarkeit und des Glanzes von Haaren, das ein spezielles Homo- oder Copolymeres, einen Polyethylenglykol und ein nicht-ionisches Tensid enthält.

Schon seit langem hat sich die Forschung auf dem Gebiet der Haarpflegemittel das Ziel gesetzt, Zubereitungen zu entwickeln, die die Formbarkeit und den Glanz des Haares erhöhen. Bisher werden hierfür Pomaden, Cremes, Gele oder Wachse verwendet, die einen hohen Anteil an Verdickern, Emulgatoren oder Konsistenzgebern aufweisen, um so das gewünschte rheologische Verhalten und die Emulsionsstabilität zu gewährleisten, die von einem zur Erhöhung der Formbarkeit und des Glanzes von Haaren geeigneten Pflegemittel verlangt werden. Nachteilig ist dabei jedoch, daß dieses Ziel mit den bisher verwendeten Haarpflegemitteln nur durch den Einsatz hoher Konzentrationen der genannten Stoffe zu erreichen ist, die einerseits zu einer Kostenerhöhung und andererseits zu einer erheblichen Belastung des Haares führen. Es stellte sich deshalb die Aufgabe, ein Haarbehandlungsmittel zu entwickeln, das diese Nachteile nicht mehr aufweist.

Das Dokument WO94/26235 offenbart eine wässrige Zusammensetzung, die ein Acrylates/Steareth-20 Methacrylat Copolymer enthält.

Gelöst wird die Aufgabe durch ein Mittel zur Erhöhung der Formbarkeit und des Glanzes von Haaren mit einem Gehalt an

(A) einem Homo- oder Copolymeren, gebildet aus mindestens einem ethylenisch ungesättigten Monomeren der allgemeinen Formel (I)

$$CH_2=CR^1R^2 \qquad (I)$$

wobei $R^1$ ausgewählt ist aus A-$(CH_2CH_2O)_x$-$R^3$ und COOH, A ausgewählt ist aus C(=O)O, C(=O)NH und $CH_2O$, x eine Zahl von 1 bis 100, vorzugsweise von 10 bis 50 ist, $R^3$ einen C1- bis C30-Alkylrest, vorzugsweise einen C8- bis C30-Alkylrest bedeutet, $R^2$ ausgewählt ist aus H, C1-C30-Alkyl und $CH_2$-$R^1$, unter der Maßgabe, daß mindestens einer der Reste $R^1$ und $R^2$ die Gruppe A-$(CH_2CH_2O)_x$-$R^3$ enthält,

(B) einem Polyethylenglykol und

(C) einem nicht-ionischen Tensid, das einen HLB-Wert unter 20 aufweist.

Durch eine geeignete Kombination dieser drei Hauptbestandteile des erfindungsgemäßen Haarbehandlungsmittels erfolgt eine unerwartet starke, synergistische Erhöhung der Viskosität und der Emulgierfähigkeit des Systems, wie sie durch die Einzelstoffe nicht erzielt werden können. Das erfindungsgemäße Mittel zeichnet sich insbesondere dadurch aus, daß es mit wasserunlöslichen Stoffen wie Kohlenwasserstoffen oder Silikonölen stabile Emulsionen bildet, wobei das rheologische Verhalten des Haarpflegemittels durch Variation des Gehaltes der oben genannten Inhaltsstoffe dem rheologischen Verhalten einer Pomade, einer Creme, eines Wachses oder eines Gels angepaßt werden kann.

Vorzugsweise ist Komponente (A) ein Copolymer, welches gebildet ist aus mindestens einem ethylenisch ungesättigten Monomeren der allgemeinen Formel (I) sowie mindestens einem ethylenisch ungesättigten Monomeren der allgemeinen Formel (II)

$$CH_2=C(R^4)COOR^5 \qquad (II)$$

wobei R4 und R5 unabhängig voneinander ausgewählt sind aus H und einer Alkylgruppe mit 1 bis 30, vorzugsweise mit 1 bis 12, insbesondere bevorzugt mit 1 bis 4 Kohlenstoffatomen.

Bevorzugt ist weiterhin, daß A ausgewählt ist aus C(=O)O und $CH_2O$, daß $R^2$ ausgewählt ist aus H und Methyl oder daß es sich bei dem Monomer der Formel (I) um ein Itaconsäurederivat handelt. Ebenfalls bevorzugt ist, daß es sich bei dem Monomer der Formel (II) um Acrylsäure, Methacrylsäure oder einen ihrer C1- bis C4-Alkylester handelt.

Geeignete Copolymere sind beispielsweise Acryl- oder Methacrylsäure/Acryl- oder Methacrylsäurepolyethoxyalkylester Copolymere (INCI-Bezeichnung: Acrylates/ Steareth-20 Methacrylate Copolymer), wie sie von der Firma Rohm und Haas/USA unter den Bezeichungen Acrysol®-22, Acrysol® ICS oder Aculyn®-22 vertrieben werden oder Acryl- oder Methacrylsäure/Polyethoxyalkylallylether Copolymere (INCI-Bezeichnung: Steareth-10 Allyl Ether/ Acrylates Copolymer), wie sie von der Firma Allied Colloids/Großbritannien unter der Bezeichnung Salcare® SC 90 vertrieben werden oder Acryl- oder Methacrylsäure/Itaconsäurepolyethoxyalkylester Copolymere (INCI-Bezeichnungen: Acrylates/Steareth-20 Itaconate Copolymer und Acrylates/Ceteth-20 Itaconate Copolymer), wie sie von der Firma National Starch/USA unter den Bezeichnungen Structure® 2001 und Structure® 3001 vertrieben werden.

Die Acryl- oder Methacrylsäuregruppen sind in den eingesetzten Polymeren vorzugsweise durch organische oder anorganische Basen, insbesondere durch primäre oder sekundäre Amine, z.B. durch Aminomethylpropanol (AMP) neutralisiert. Das Polymer ist in dem Haarbehandlungsmittel in einer Menge von 0,01 bis 10, vorzugsweise in einer Menge von 0,05 bis 3 Gew.-% enthalten.

Die in dem erfindungsgemäßen Mittel enthaltenen Polyethylenglykole sind bei Raumtemperatur flüssig, wachsartig oder fest. Sie haben im allgemeinen ein Molekulargewicht von über 500, vorzugsweise zwischen 20.000 und 3.600.000 und können unter den Handelsbezeichnungen Lipoxol® 1000 von der Firma Hüls, unter der Bezeichnung Pluracol® E 4000 von der Firma BASF, unter der Bezeichnung Polyglykol von der Firma Hoechst, unter der Bezeichnung Upiwax® 20.000 von der Firma UPI oder unter den Typenbezeichnungen Polyox® WSR-301 und Polyox® WSR-N-60 K von der Union Carbidebezogen werden. Das Polyethylenglykol wird in einer Menge von 0,01 bis 50, vorzugsweise von 0,1 bis 20 Gew.-% eingesetzt.

Als weiteren wichtigen Bestandteil enthält das erfindungsgemäße Haarpflegemittel ein nicht-ionisches Tensid. Bevorzugt sind dabei ethoxylierte Tenside, wobei die Anzahl der Ethylenoxyd-Einheiten zwischen 1 bis 1000, bevorzugt jedoch zwischen 1 bis 300 liegt. Bevorzugt werden Fettalkoholethoxylate, Fettaminethoxylate, Fettsäurealkanolamidethoxylate und Fettsäureesterethoxylate. Beispiele für geeignete Fettalkoholethoxylate sind oxethylierter Lauryl-, Tetradecyl-, Cetyl-, Oleyl- oder Stearylalkohol, die allein oder im Gemisch eingesetzt werden können, sowie Fettalkohole von oxethyliertem Lanolin oder oxethyliertes Lanolin. Auch die ethoxylierten Fettalkohole, die unter der Typenbezeichnung Dehydol® von der Firma Henkel oder unter der Typenbezeichnung Brij® von der Firma ICI Surfactants vertrieben werden, sind für das erfindungsgemäße Haarbehandlungsmittel geeignet.

Unter den Fettsäureesterethoxylaten sind vor allem Diglyceridethoxylate zu nennen wie das von der ICI Surfactants unter dem Handelnamen Arlatone® G vertriebene, mit 25 Ethylenoxid-Einheiten ethoxylierte Rizinusöl mit dem INCI-Namen PEG-25 Hydrogenated Castor Oil, das von der BASF unter dem Handelnamen Cremophor® El vertriebene, mit 35 Ethylenoxid-Einheiten ethoxylierte Rizinusöl mit dem INCI-Namen PEG-35 Castor Oil, das von der BASF unter dem Handelsnamen Cremophor® RH 410 vertriebene, mit 40 Ethylenoxid-Einheiten ethoxylierte, hydrierte Rizinusöl mit dem INCI-Namen PEG-40 Hydrogenated Castor Oil, und die von der Firma Witco Surfactants unter dem Namen Rewoderm® LI vertriebenen Rohstoffe zu nennen.

Weiterhin können die als nicht-ionische Tenside bekannten ethoxylierten Fettsäurezuckerester, insbesondere der ethoxylierte Sorbitanfettsäureester, aber auch nicht ethoxylierte Tenside, wie die Fettsäurezuckerester, die von der Firma ICI Surfactants unter dem Handelsnamen Tween® und Arlacel® vertrieben werden sowie die Alkylpolyglycoside, die von der Firma Henkel unter dem Handelsnamen Plantaren® oder Plantacare® oder von der Firma Seppic unter dem Handelsnamen Oramix® vertrieben werden, für die erfindungsgemäße kosmetische Zubereitung eingesetzt werden.

Generell läßt sich sagen, daß nicht-ionische Tenside für das erfindungsgemäße Haarbehandlungsmittel geeignet sind, wenn sie einen HLB-Wert unter 20, vorzugsweise einen HLB-Wert unter 12 und ganz besonders bevorzugt unter 9 aufweisen. Das Tensid wird in einer Menge von 0,01 bis 20 Gewichtsprozent eingesetzt.

Zusätzlich zu den vorstehend genannten drei Substanzklassen kann das erfindungsgemäße Mittel weitere Bestandteile enthalten. An erster Stelle sind hier natürliche oder synthetische, filmbildende und damit haarfestigende Polymere zu nennen, die aus den Klassen der anionischen, kationischen oder amphoteren oder nicht-ionischen Polymeren ausgewählt werden. Üblicherweise werden diese Polymere in Mengen von 0,01 bis 30 Gew.-%, vorzugsweise in Mengen von 0,5 bis 10 Gew.-% eingesetzt, wobei jedoch bei der Anwendung von kationischen Polymeren die bevorzugte Konzentration zwischen 0,01 und 2 Gew.-% liegt.

Unter filmbildenden, haarfestigenden Polymeren werden solche Polymere verstanden, die in einer 0,1 bis 5%igen wässrigen, alkoholischen oder wässrig-alkoholischen Lösung angewendet in der Lage sind, auf dem Haar einen Polymerfilm abzuscheiden und auf diese Weise das Haar zu festigen.

Geeignete natürliche filmbildende Polymere mit haarfestigender Wirkung sind niedermolekulares Chitosan mit einem Molekulargewicht von 30.000 bis 70.000 g/mol, welches beispielsweise von der Firma Kyowa Oil & Fat, Japan vertrieben wird. Desweiteren können verschiedene Saccharidtypen verwendet werden wie Polysaccharide oder Gemische aus Oligo-, Mono- und Disacchariden, welche beispielsweise unter dem Handelsnamen C-PUR® von der Firma Cerestar, Brüssel, vertrieben werden. Weitere geeignete, natürliche Polymere sind chinesisches Balsamharz und Cellulosederivate, z.B. Hydroxypropylcellulose mit einem Molekuklargewicht von 30.000 bis 50.000 g/mol, welche beispielsweise unter der Handelsbezeichnung Nisso Sl® von der Firma Lehmann & Voss, Hamburg, vertrieben wird. Ein weiteres natürliches Polymer ist Schellack, wie er beispielsweise unter der Handelsbezeichnung Schellack MHP 210 von der Fa. Pennig, Hamburg, vertrieben wird. Schellack kann in neutralisierter Form und unneutralisiert zum Einsatz kommen.

Geeignete synthetische filmbildende, anionische Polymere sind z.B. Crotonsäure-Vinylacetat-Copolymere, die beispielsweise in Form einer 60%igen Lösung in Isopropanol/ Wasser unter der Handelsbezeichnung Aristoflex® von Hoechst vertrieben werden. Weitere geeignete anionische Polymere sind z.B. Terpolymere aus Acrylsäure, Ethylacrylat und N-t-Butylacrylaminde wie sie unter dem Handelsnamen Ultrahold® und Ultrahold® strong der BASF vertrieben werden.

Von den kationischen Polymeren, die in dem erfindungsgemäßen Mittel enthalten sein können, sind z.B. Polyvinylpyrrolidon/Dimethylaminoethylmethacrylat-Polymere, das unter der Handelsbezeichnung Gafquat® 755 N von der Firma Gaf Co., New York, vertrieben wird. Weitere kationische Polymere sind beispielsweise das von der BASF unter dem Handelsnamen Luviquat® HM 550 vertriebene Copolymer aus Polyvinylpyrolidon und Imidazoliminmethochlorid, das von der Firma Calgon, Pittsburgh, unter dem Handelsnamen Merquat® Plus 3300 vertriebene Terpolymer aus Dimethyldiallylammoniumchlorid, Natriumacrylat und Acrylamid, das von der Firma ISP/USA unter dem Handelsnamen Gaffix® VC 713 vertriebene Terpolymer aus Vinylpyrrolidon, Dimethylaminoethylmethacrylat und Vinylcaprolactam, das von der Firma Amerchol, Edison/USA, unter dem Handelsnamen Polymer IR® vertriebene quaternierte Ammoniumsalz der Hydroxyethylcellulose und einem Trimethylammonium-substituierten Epoxid, das von der Firma Gaf unter dem Handelsnamen Gafquat® HS 100 vertriebene Vinylpyrrolidon/Methacrylamidopropyltrimethylammoniumchlorid Copolymer und das von der Firma Goldschmidt, Essen, unter dem Handelsnamen Abil® Quat 3272 vertriebene diquaternäre Polydimethylsiloxan.

Geeignete amphotere Polymere sind z.B. Copolymere aus Octoylacrylamid, t-Butylaminoethylmethacrylat und zwei oder mehr Monomeren, bestehend aus Acrylsäure, Methacrylsäure oder deren Estern, wie sie z.B. unter dem Handelsnamen Resyn® 28-4910 oder Amphomer® LV-71 der Firma National Starch, USA, erhältlich sind.

Geeignete synthetische, nicht-ionische filmbildende, haarfestigende Polymere sind Homopolymere des Vinylpyrrolidons, die beispielsweise unter den Handelsbezeichnungen Luviskol® K von der BASF oder PVP-K von der Firma ISP, Wayne vertrieben werden, sowie Homopolymere des N-Vinylformamids, die beispielsweise unter der Handelsbezeichnung PVF von der Firma National Starch vertrieben werden. Weitere geeignete synthetische filmbildende, nichtionische, haarfestigende Polymere sind z.B. Copolymerisate aus Vinylpyrrolidon und Vinylacetat, die beispielsweise unter den Handelsbezeichnungen Luviskol® VA von der BASF vertrieben werden; Terpolymere aus Vinylpyrrolidon, Vinylacetat und vinylpropionat, die beispielsweise unter der Handelsbezeichnung Luviskol® VAP der BASF vertrieben werden; Polyacrylamide, die beispielsweise unter den Handelsbezeichnungen Akyponine® P 191 von der Firma CHEM-Y, Emmerich, oder Sepigel® 305 von der Firma Seppic vertrieben werden; Polyvinylalkohole, die beispielsweise unter den Handelsbezeichnungen Elvanol® von Du Pont oder Vinol® 523/540 von der Firma Air Products vertrieben werden sowie Polyethylenglykol/ Polypropylenglykol-Copolymere, die beispielsweise unter den Handelsbezeichnungen Ucon® der Union Carbide vertrieben werden.

Außerdem können auch Polymere mit verdickender Wirkung eingesetzt werden, wenn sie mit den übrigen Bestandteilen des erfindungsgemäßen Mittels verträglich sind.

Von den Verdickern, die in dem erfindungsgemäßen Mittel enthalten sein können, sind Homopolymere der Acrylsäure mit einem Molekulargewicht von 2.000.000 bis 6.000.000 zu nennen, die beispielsweise von der Firma BF Goodrich, Cleveland, unter der Handelsbezeichnung Carbopol® vertrieben werden. Als weitere Verdicker kann das erfindungsgemäße Mittel ein Acrylsäurehomopolymer mit einem Molekulargewicht von 4.000.000 enthalten, das beispielsweise von Goodrich unter der Handelsbezeichnung Carbopol® 940 vertrieben wird. Weitere Verdicker sind beispielsweise die von Goodrich unter dem Handelsnamen Carbopol® ETD 2001 oder von der Firma Protex/Frankreich unter dem Handelsnamen Modarez V 600 PX vertriebene Acrylsäurehomopolymer, das von Hoechst unter dem Handelsnamen Hostacerin® PN 73 vertriebene Polymer aus Acrylsäure und Acrylamid (Natriumsalz mit einem Molekulargewicht von 2.000.000 bis 6.000.000) und das von der Firma Alban Müller, Montreuil, unter dem Handelsnamen Amigel® vertriebene Sclerotium Gum. Besonders bevorzugt sind die Copolymeren der Acrylsäure oder der Methacrylsäure, wie sie z.B. unter dem Handelsnamen Carbopol® 1342 oder Pemulen® TR1 von Goodrich vertrieben werden.

Des weiteren kann das erfindungsgemäße Mittel wasserlösliche oder wasserunlösliche Silikonverbindungen in einer Konzentration von 0,01 bis 50 Gewichtsprozent, bevorzugt in einer Konzentration von 0,1 bis 5 Gewichtsprozent enthalten. Besonders bevorzugt sind dabei flüchtige und nichtflüchtige Cyclomethicone und Dimethicone sowie Dimethicon-Copolyole. Beispiele sind: Polydimethylsiloxan (INCI: Dimethicon), $\alpha$-Hydro-$\omega$-hydroxypolyoxydimethylsilylen (INC:Dimethiconol), cyclisches Dimethylpolysiloxan (INCI:Cyclomethicon), Trimethyl-(octadecyloxy) silan (INCI:Stearoxytrimethylsilan), Dimethylsiloxan-Glykol-Copolymer (INCI:Dimethicon Copolyol), Dimethylsiloxanaminoalkylsiloxan-Copolymer mit Hydroxyendgruppen (INCI:Amodimethicon), Monomethylpolysiloxan mit Laurylseitenketten und Polyoxyethylen- und/oder Polyoxypropylenendketten, (INCI:Laurylmethicon Copolyol), Dimethylsiloxan-glykol-copolymeracetat (INCI:Dimethiconcopolyol Acetat) Dimethylsiloxanaminoalkylsiloxan-Copolymer mit Trimethylsilylendgruppen (INCI:Trimethylsilylamodimethicon). Bevorzugte Silikonpolymere sind: Dimethicone, welche beispielsweise von der Firma Wacker, München, unter der Handelsbezeichnung Siloxane F-221 oder von der Firma-Dow Corning Europe, Brüssel, unter der Handelsbezeichnung Dow Corning Fluid 200/0,65 cs vertrieben werden; Cyclomethicone, die beispielsweise unter den Handelsbezeichnungen Dow Corning 244 Fluid von der Firma Dow Corning Europe oder Abil® K4 von der Firma Goldschmidt vertrieben werden; Dimethiconole, die beispielsweise unter den Handelsbezeichnungen Silicone Fluid F-212 von der Firma Wacker oder Unisil® SF-R von der Firma UPI vertrieben werden.

Die vorstehend in Klammern angegebenen Bezeichnungen entsprechen der INCI Nomenklatur (International Cosmetic Ingredients), wie sie zur Kennzeichnung kosmetischer Wirk- und Hilfsstoffe bestimmt sind.

Auch Mischungen von Silikonpolymeren sind geeignet, wie z.B. eine Mischung aus Dimethicon und Dimethiconol, die beispielsweise unter der Handelsbezeichnung Dow Corning 1403 Fluid von der Firma Dow Corning Europe vertrieben wird.

Weitere geeignete Silikonpolymere sind Dimethicon Copolyole, weche unter den Handelsnamen Surfactant 193 von der Firma Dow Corning Europe oder Silwet® L von der Union Carbide vertrieben werden; Amodimethicone, die beispielsweise unter den Handelsnamen Sandoperm® FE von Sandoz oder SM 2059 von General Electric/USA vertrieben werden; Laurylmethicon Copolyol, das unter der Handelsbezeichnung Dow Corning Q2-5200 von der Firma Dow Corning Europe vertrieben wird; Trimethylsilylamodimethicone, die unter den Handelsbezeichnungen Dow Corning Q2-8220 von der Firma Dow Corning Europe oder Silicone Fluid F-801 von der Firma Wacker vertrieben werden; Dimethicon Copolyol Acetate, die unter den Handelsbezeichnungen Silicone Fluid VP oder Belsil® DMC 6033 von der Firma Wacker vertrieben werden und Trimethyl-(octadecyloxy)silane (INCI:Stearoxytrimethylsilane), die beispielsweise unter der Handelsbezeichnung Dow Corning 580 WAX von der Firma Dow Corning Europe vertrieben werden.

Dem Haarbehandlungsmittel können Wasser oder Alkohol-Wassermischungen zugesetzt werden. Als Alkohole können insbesondere die für kosmetische Zwecke üblicherweise verwendeten niederen Alkohole mit 1 bis 4 Kohlenstoffatomen, wie Ethanol und Isopropanol enthalten sein.

Desweiteren können Lösungsmittel oder ein Gemisch aus Lösungsmitteln mit einem Siedepunkt unter 600 °C in einer Menge von 0,01 bis 90 Gew.-%, bevorzugt von 2 bis 50 Gew.-% eingesetzt werden. Besonders geeignet sind dabei Propylenglykole sowie Glycerine.

Das erfindungsgemäße Mittel kann zusätzlich wasserunlösliche Lösungsmittel oder Substanzen enthalten. Beispiele für wasserunlösliche Lösungsmittel sind unverzweigte oder verzweigte Kohlenwasserstoffe wie Pentan, Hexan, Isopentan und zyklische Kohlenwasserstoffe wie Cyclopentan und Cyclohexan. Besonders bevorzugt sind Paraffine sowie Isododecan.

Selbstverständlich können die erfindungsgemäßen Mittel auch weitere übliche kosmetische Zusätze, wie nichtfestigende, nicht-ionische Polymere, nichtfestigende, anionische Polymere und nicht festigende, natürliche Polymere sowie deren Kombination in einer Menge von vorzugsweise 0,01 bis 50 Gew.-%; Parfümöle in einer Menge von vorzugsweise 0,01 bis 5 Gew.-%; Trübungsmittel wie z.B. Ethylenglykoldistearat, Styrol PVP-Copolymere, Polystyrole, in einer Menge von vorzugsweise 0,01 bis 5 Gew.-%; Netzmittel, Tenside oder Emulgatoren mit oder ohne Waschaktivität aus den Klassen der anionischen, kationischen, amphoteren oberflächenaktiven Substanzen wie Fettalkoholsulfate, Fettalkoholethersulfate, Fettsäurealkanolamide in einer Menge von vorzugsweise 0,1 bis 30 Gew.-%; ferner Feuchthaltemittel, Farbstoffe, Lichtschutzmittel, Antioxidantien, Glanzgeber und Konservierungsstoffe in einer Menge von vorzugsweise 0,01 bis 10 Gew.-% enthalten. Des weiteren kann das erfindungsgemäße Mittel auch Konsistenzgeber wie sie üblicherweise für Cremes eingesetzt werden, enthalten. Beispiele sind die von Henkel unter der Handelsbezeichnung Lanette verkauften Verbindungen.

Die überraschenden Eigenschaften der erfindungsgemäßen Mischung können, wie die nachfolgenden Beispiele zeigen, insbesondere anhand von Viskositätsmessungen deutlich nachgewiesen werden.

**Beispiele**

Es wurden Viskositätsmessungen mit dem Rotationsviskosimeter Haake VT-550 durchgeführt und Viskositäten bei 25° C in mPas bei 100 s$^{-1}$ ermittelt. Hierfür wurden Mischungen der Lösungen 1, 2 und 3 mit unterschiedlichen nicht-ionischen Tensiden versetzt und der jeweilige Viskositätsanstieg beobachtet.

Lösung 1:    3.0% Acrysol® 22 in Wasser; neutralisiert mit Aminomethylpropanol auf pH 7
Lösung 2:    0,5% Polyox® WSR-301 in Wasser/Propylenglykol (9/1)
Lösung 3:    Polyglykol 200 0,5% in Wasser

Tabelle 1:

| Viskositätsanstieg bei Zugabe von ethoxylierten Tensiden zu einer Mischung von Acrysol® 22 mit einem hochmolekularen Polyethylenglykol | | | | | | | |
|---|---|---|---|---|---|---|---|
| | V1 | V2 | V3 | V4 | V5 | V6 | V7 |
| Lösung 1 | 50 | 48.75 | 48.75 | 48.75 | 48.75 | 48.75 | 48.75 |
| Lösung 2 | 50 | 48.75 | 48.75 | 48.75 | 48.75 | 48.75 | 48.75 |

Tabelle 1: (fortgesetzt)

| Viskositätsanstieg bei Zugabe von ethoxylierten Tensiden zu einer Mischung von Acrysol® 22 mit einem hochmolekularen Polyethylenglykol | | | | | | | |
|---|---|---|---|---|---|---|---|
| | V1 | V2 | V3 | V4 | V5 | V6 | V7 |
| Cremophor® RH 410 | | 2.5 | | | | | |
| Cremophor® EL | | | 2.5 | | | | |
| Dehydol® LS4 | | | | 2.5 | | | |
| Brij® 30 | | | | | 2.5 | | |
| Tween® 40 | | | | | | 2.5 | |
| Rewoderm® LI S 80 | | | | | | | 2,5 |
| Viskosität | 192 | 704 | 676 | 3255 | 3581 | 297 | 369 |

Tabelle 2:

| Viskositätsanstieg bei Zugabe von ethoxylierten Tensiden zu einer Mischung von Acrysol® 22 mit einem niedrigmolekularen Polyethylenglykol | | | | |
|---|---|---|---|---|
| | V8 | V9 | V10 | V11 |
| Lösung 1 | 50 | 48.75 | 48.75 | 48.75 |
| Lösung 3 | 50 | 48.75 | 38.75 | 48.75 |
| Cremophor® RH410 | | 2.75 | | |
| Dehydol® LS 4 | | | 2.75 | |
| Rewoderm® LI S80 | | | | 2.75 |
| Viskosität | <100 | 645 | 4572 | 329 |

Tabelle 3:

| Viskositätsanstieg bei Zugabe von nicht-ethoxylierten Tensiden zu einer Mischung von Acrysol® 22 mit einem hochmolekularen Polyethylenglykol | | |
|---|---|---|
| | V12 | V13 |
| Lösung 1 | 50 | 48.75 |
| Lösung 2 | 50 | 48.75 |
| Plantacare® 818 UP | | 2.75 |
| Viskosität | <192 | 1132 |

Tabelle 4:

| Viskositätsanstieg bei Zugabe eines nicht-ethoxylierten Tensids zu einer Mischung von Acrysol® 22 mit einem niedrigmolekularen Polyethylenglykol | | |
|---|---|---|
| | V12 | V13 |
| Lösung 1 | 50 | 48.75 |
| Lösung 3 | 50 | 48.75 |

Tabelle 4:   (fortgesetzt)

| Viskositätsanstieg bei Zugabe eines nicht-ethoxylierten Tensids zu einer Mischung von Acrysol® 22 mit einem niedrigmolekularen Polyethylenglykol | | |
|---|---|---|
| | V12 | V13 |
| Plantacare 818 UP | | 2.75 |
| Viskosität | <100 | 1132 |

Dieser extreme und unerwartete Viskositätsanstieg macht die erfindungsgemäße Mischung geeignet zur Herstellung der nachfolgenden Rezepturen:

**Beispiel 1: Gel mit starker Festigung und nichtionischem Polymer**

| | |
|---|---|
| 92.6 g | 2% Acrysol® 22 in Wasser, mit Base auf pH 7 eingestellt |
| 5.0 g | Luviskol® K80 |
| 0.4 g | Polyox® WSR-301 |
| 2.0 g | Cremophor® RH 410 |
| 100.00 g | |

**Beispiel 2: Gel mit starker Festigung und amphoterem Polymer**

| | |
|---|---|
| 92.3 g | 1,5% Acrysol® 22 in Wasser, mit Base auf pH 7 eingestellt |
| 5.0 g | Amphomer®, neutralisiert mit AMP |
| 0.7 g | Polyox® WSR N-10 |
| 2.0 g | Cremophor® RH 455 |
| 100.00 g | |

**Beispiel 3: Gel mit starker Festigung und anionischem Polymer**

| | |
|---|---|
| 92.4 g | 1.5% Acrysol® ICS-1 in Wasser, mit Base auf pH 7eingestellt |
| 5.0 g | Luviset® CAP ( Copolymeres aus Vinylacetat, Crotonsäure und Vinylpropionsäure), neutralisiert mit AMP |
| 0.6 g | Polyox® WSR N-80 |
| 2.0 g | Cremophor® EL |
| 100.00 g | |

**Beispiel 4: Gel mit starker Festigung und nichtionischem sowie kationischem Polymer**

| | |
|---|---|
| 92.6 g | 1.5% Structure® 2001 in Wasser, mit Base auf pH 7 eingestellt |
| 4.9 g | Luviskol® VA 64 |
| 0.1 g | Gafquat® 755 N |
| 0.4 g | Polyox® WSR N-3000 |
| 2.0 g | Dehydol® LS 4 |
| 100.00 g | |

**Beispiel 5: Gel mit Festigung und Schutz vor Sonne**

| | |
|---|---|
| 92.2 g | 1.5% Acrysol® 22 in Wasser, mit Base auf pH 7 eingestellt |
| 5.0 g | Luviskol® VA 64 |
| 0.5 g | Uvinul® P25 ( p-Aminobenzoesäure mit 25 Mol Ethylenglykol) |
| 0.3 g | Polyox® WSR-205 |
| 2.0 g | Dehydol® LS 4 |
| 100.00g | |

**Beispiel 6: Gel mit Wet-Effekt**

| | |
|---|---|
| 82.8 g | 1% Acrysol® 22 in Wasser, mit Base auf pH 7 eingestellt |
| 5.0 g | Luviskol® K 90 |
| 0.2 g | Polyox® WSR N-60 K |
| 2.0 g | Brij® 30 |
| 10.0 g | Glycerin |
| 100.00g | |

**Beispiel 7: Schnelltrocknendes Gel mit Ethanol**

| | |
|---|---|
| 61.0 g | 2.5% Salcare® SC90 in Wasser, mit Base auf pH 7 eingestellt |
| 5.0 g | Luviskol® K80 |
| 2.0 g | Polyglykol 3000 |
| 2.0 g | Tween® 40 |
| 30.0 g | Ethanol |
| 100.00 g | |

**Beispiel 8: Gel mit leichter Festigung und Silikonöl als Glanzgeber**

| | |
|---|---|
| 83.8 g | 1.5% Structure® 3001 in Wasser, mit Base auf pH 7 eingestellt |
| 3.0g | Silikonöl AK 500 |
| 0.2 g | Polyox® WSR-301 |
| 3.0 g | Cremophor® RH 410 |
| 10.0 g | Glycerin |
| 100.00 g | |

**Beispiel 9: Pomade mit leichter Festigung**

| | |
|---|---|
| 77.8 g | 1% Salcare SC90 in Wasser, mit Base auf pH 7 eingestellt |
| 10.0 g | Castor Oil PEG 25 |
| 0.2 g | Polyglykol 35000 |
| 2.0 g | Rewoderm® LI S 80 |
| 10.0 g | Glycerin |
| 100.00 g | |

**Beispiel 10: Pomade mit Festigung**

| | |
|---|---|
| 69.8 g | 1% Acrysol® ICS-I in Wasser, mit Base auf pH 7 eingestellt |
| 10.0 g | Castor Oil PEG 25 |
| 3.0 g | Luviskol® K 60 |
| 5.2 g | Polyglykol 200 |
| 2.0 g | Rewoderm® LI S 80 |
| 10.0 g | Glycerin |
| 100.00 g | |

**Beispiel 11: Pomade mit leichter Festigung und Langzeitglanz**

| | |
|---|---|
| 71.0 g | 1% Structure® 2001 in Wasser, mit Base auf pH 7 eingestellt |
| 3.0 g | Kamol® ID (Isododecan) |
| 10.0 g | Castor Oil PEG 25 |
| 1.0 g | PEG-14000 |
| 2.0 g | Emulgin® L (PPG-1-PEG-9-Laurylglykolether) |
| 10.0 g | Glycerin |
| 3.0 g | Dow Corning Q2-1403 |
| 100.00 g | |

**Beispiel 12: Form- und Glanzcreme**

| | |
|---|---|
| 77.8 g | 1% Structure® 3001 in Wasser, mit Base auf pH 7 eingestellt |
| 10.0 g | Paraffinium perliquidum |
| 0.2 g | Polyox® WSR-301 |
| 2.0 g | Cremophor® RH 410 |
| 10.0 g | Glycerin |
| 100.00 g | |

**Beispiel 13: Form- und Glanzcreme mit Siliconöl**

| | |
|---|---|
| 77.2 g | 1% Acrysol® 22 in Wasser, mit Base auf pH 7 eingestellt |
| 5.0 g | Surfactant 193 |
| 5.8 g | Polyglykol 200 |
| 2.0 g | Cremophor® EL |
| 10.0 g | Glycerin |
| 100.00 g | |

**Beispiel 14: Festigende Creme ohne Belastung**

| | |
|---|---|
| 87.8 g | 1% Acrysol® 22 in Wasser, mit Base auf pH 7 eingestellt |
| 5 g | Luviskol® K50 |
| 0.2 g | Polyox® WSR-301 |
| 2.0 g | Cremophor® RH 410 |
| 5 g | Kamol® ID ( Isododecan ) |
| 100.00 g | |

**Beispiel 15: Form- und Glanzcreme ohne Belastung**

| 76,8 g | 1% Acrysol® 22 in Wasser, mit Base auf pH 7 eingestellt |
|---|---|
| 10 g | Dow Corning Fluid 200 /(0.65 mPas) |
| 0.2 g | Polyox® WSR N-60K |
| 2.0 g | Dehydol® LS 4 |
| 1.0 g | Cetiol® HE |
| 100.00 g | |

**Beispiel 16: Form- und Glanzcreme mit Schutz vor Sonne**

| 86.3 g | 1% Salcare® SC90 in Wasser, mit Base auf pH 7 eingestellt |
|---|---|
| 10 g | Dow Corning Fluid 200 (0.65 mPas) |
| 0.2 g | Polyglykol 1000 |
| 2.0 g | Dehydol® LS 4 |
| 1.0 g | Cetiol® HE |
| 0.5 g | Uvinul® P25 ( p-Aminobenzoesäure mit 25 Mol Ethylenglykol ) |
| 100.00 g | |

**Beispiel 17: Glanzwachs mit Silikonöl**

| 71.4 g | 2.5% Acrysol® 22 in Wasser, mit Base auf pH 7 eingestellt |
|---|---|
| 3.0 g | Luviskol® K 120 |
| 10.0 g | Abil® K4 |
| 0.4 g | Polyox® WSR-301 |
| 5.4 g | Cremophor® RH 410 |
| 10.0 g | Glycerin |
| 100.00 g | |

**Beispiel 18: Glanzwachs mit Paraffinöl**

| 71.4 g | 2.5% Acrysol® 22 in Wasser, mit Base auf pH 7 eingestellt |
|---|---|
| 3.0 g | Luviskol® K 120 |
| 10.0 g | Paraffinum perliquidum |
| 0.4 g | Polyox® WSR-301 |
| 5.0 g | Cremophor® RH 410 |
| 10.0 g | Glycerin |
| 100.00 g | |

**Beispiel 19: Stylingwachs**

| | |
|---|---|
| 74.8 g | 2% Acrysol® 22 in Wasser, mit Base auf pH 7 eingestellt |
| 3 g | Luviskol® K 120 |
| 10.0 g | Abil® K4 |
| 0.2 g | Polyox® WSR-301 |
| 2.0 g | Cremophor® RH 410 |
| 10.0 g | Glycerin |
| 100.00 g | |

**Patentansprüche**

1.  Mittel zur Erhöhung der Formbarkeit und des Glanzes von Haaren mit einem Gehalt an

    (A) einem Homo- oder Copolymeren, gebildet aus mindestens einem ethylenisch ungesättigten Monomeren der allgemeinen Formel (I)

    $$CH_2=CR^1R^2 \qquad \text{(I)}$$

    wobei $R^1$ ausgewählt ist aus A-$(CH_2CH_2O)_x$-$R^3$ und COOH, A ausgewählt ist aus C(=O)O, C(=O)NH und $CH_2O$, x eine Zahl von 1 bis 100 ist, $R^3$ einen C1- bis C30-Alkylrest bedeutet, $R^2$ ausgewählt ist aus H, C1-C30-Alkyl und $CH_2$-$R^1$, unter der Maßgabe, daß mindestens einer der Reste $R^1$ und $R^2$ die Gruppe A-$(CH_2CH_2O)_x$-$R^3$ enthält,
    (B) einem Polyethylenglykol und
    (C) einem nicht-ionischen Tensid, das einen HLB-Wert unter 20 aufweist.

2.  Mittel nach Anspruch 1, dadurch gekennzeichnet, daß Komponente (A) ein Copolymer ist, gebildet aus mindestens einem ethylenisch ungesättigten Monomeren der allgemeinen Formel (I) sowie mindestens einem ethylenisch ungesättigten Monomeren der allgemeinen Formel (II)

    $$CH_2=C(R^4)COOR^5 \qquad \text{(II)}$$

    wobei R4 und R5 unabhängig voneinander ausgewählt sind aus H und C1- bis C30-Alkyl.

3.  Mittel nach einem der vorstehenden Ansprüche, dadurch gekennzeichnet, daß A ausgewählt ist aus C(=O)O und $CH_2O$, $R^2$ ausgewählt ist aus H und Methyl und $R^3$ einen C8- bis C30-Alkylrest bedeutet.

4.  Mittel nach Anspruch 1 oder 2, dadurch gekennzeichnet, daß es sich bei dem Monomer der Formel (I) um ein Itaconsäurederivat handelt.

5.  Mittel nach einem der vorstehenden Ansprüche, dadurch gekennzeichnet, daß das Monomer der Formel (II) Acrylsäure, Methacrylsäure oder einer deren C1- bis C4-Alkylester ist.

6.  Mittel nach einem der vorstehenden Ansprüche, dadurch gekennzeichnet, daß Komponente (A) ausgewählt ist aus Acryl- oder Methacrylsäure/Acryl- oder Methacrylsäurepolyethoxyalkylester Copolymeren, Acryl- oder Methacrylsäure/Polyethoxyalkylallylether Copolymeren und Acryl- oder Methacrylsäure/Itaconsäurepolyethoxyalkylester Copolymeren.

7.  Mittel nach einem der vorstehenden Ansprüche, dadurch gekennzeichnet, daß freie Carbonsäuregruppen der Komponente (A) in neutralisierter Form vorliegen.

8.  Mittel nach einem der vorstehenden Ansprüche, dadurch gekennzeichnet, daß es Komponente (A) in einer Menge von 0,01 bis 10 Gewichtsprozent enthält.

9.  Mittel nach einem der vorstehenden Ansprüche, dadurch gekennzeichnet, daß es das Polyethylenglykol in einer Menge von 0,01 bis 50 Gewichtsprozent enthält.

10. Mittel nach einem der vorstehenden Ansprüche, dadurch gekennzeichnet, daß es das nicht-ionische Tensid in einer Menge von 0,01 bis 20 Gewichtsprozent enthält.

11. Mittel nach einem der vorstehenden Ansprüche, dadurch gekennzeichnet, daß das Polyethylenglykol ein Molekulargewicht von über 500, vorzugsweise von 20.000 bis 3.600.000 aufweist.

12. Mittel nach einem der vorstehenden Ansprüche, dadurch gekennzeichnet, daß es als nicht-ionisches Tensid ein Fettalkoholethoxylat, ein Fettaminethoxylat, ein Fettsäurealkanolamidethoxylat oder ein Fettsäureesterethoxylat enthält.

13. Mittel nach einem der vorstehenden Ansprüche, dadurch gekennzeichnet, daß es zusätzlich ein natürliches oder synthetisches, anionisches, kationisches, amphoteres oder nicht-ionisches, haarfestigendes Polymeres in einer Menge von 0,01 bis 30 Gewichtsprozent enthält.

14. Mittel nach einem der vorstehenden Ansprüche, dadurch gekennzeichnet, daß es zusätzlich eine wasserlösliche oder wasserunlösliche Silikonverbindung in einer Menge von 0,01 bis 30 Gewichtsprozent enthält.

15. Mittel nach einem der vorstehenden Ansprüche, dadurch gekennzeichnet, daß es Wasser und/oder einen Alkohol mit 1 bis 4 Kohlenstoffatomen, vorzugsweise Ethanol oder isopropanol enthält.


**Claims**

1.  Agent for increasing the shapability and the sheen of hair, containing

    (A) a homopolymer or copolymer, formed from at least one ethylenically unsaturated monomer of general formula (I)

    $$CH_2=CR^1R^2 \qquad\qquad (I),$$

    wherein $R^1$ is selected from A-$(CH_2CH_2O)_x$-$R^3$ and COOH, A is selected from C(=O)O, C(=O)NH and $CH_2O$, x is a number from 1 to 100, $R^3$ represents a C1 to C30 alkyl radical, $R^2$ is selected from H, C1 to C30 alkyl and $CH_2$-$R^1$, provided that at least one of the radicals $R^1$ and $R^3$ contains the group A-$(CH_2CH_2O)_x$-$R^3$,
    (B) a polyethylene glycol and
    (C) a non-ionic surfactant which has an HLB value of less than 20.

2.  Agent according to claim 1, characterised in that component (A) is a copolymer, formed from at least one ethylenically unsaturated monomer of general formula (I) and at least one ethylenically unsaturated monomer of general formula (II)

    $$CH_2=C(R^4)COOR^5 \qquad\qquad (II),$$

    wherein R4 and R5 are selected independently of one another from H and C1 to C30 alkyl.

3.  Agent according to either of the preceding claims, characterised in that A is selected from C(=O)O and $CH_2O$, $R^2$ is selected from H and methyl and $R^3$ represents a C8 to C30 alkyl radical.

4.  Agent according to claim 1 or claim 2, characterised in that the monomer of formula (I) is an itaconic acid derivative.

5.  Agent according to any one of the preceding claims, characterised in that the monomer of formula (II) is acrylic acid, methacrylic acid or one of the C1 to C4 alkyl esters thereof.

6. Agent according to any one of the preceding claims, characterised in that component (A) is selected from acrylic or methacrylic acid/acrylic or methacrylic acid polyethoxyalkyl ester copolymers, acrylic or methacrylic acid/polyethoxyalkyl allyl ether copolymers and acrylic or methacrylic acid/itaconic acid polyethoxyalkyl ester copolymers.

7. Agent according to any one of the preceding claims, characterised in that free carboxylic acid groups of component (A) are present in neutralised form.

8. Agent according to any one of the preceding claims, characterised in that it contains component (A) in a quantity of from 0.01 to 10 percent by weight.

9. Agent according to any one of the preceding claims, characterised in that it contains the polyethylene glycol in a quantity of from 0.01 to 50 percent by weight.

10. Agent according to any one of the preceding claims, characterised in that it contains the non-ionic surfactant in a quantity of from 0.01 to 20 percent by weight.

11. Agent according to any one of the preceding claims, characterised in that the polyethylene glycol has a molecular weight which is greater than 500 and preferably from 20,000 to 3,600,000.

12. Agent according to any one of the preceding claims, characterised in that it contains as the non-ionic surfactant a fatty alcohol ethoxylate, a fatty amine ethoxylate, a fatty acid alkanolamide ethoxylate or a fatty acid ester ethoxylate.

13. Agent according to any one of the preceding claims, characterised in that it additionally contains a natural or synthetic, anionic, cationic, amphoteric or non-ionic hair-setting polymer in a quantity of from 0.01 to 30 percent by weight.

14. Agent according to any one of the preceding claims, characterised in that it additionally contains a watersoluble or water-insoluble silicone compound in a quantity of from 0.01 to 30 percent by weight.

15. Agent according to any one of the preceding claims, characterised in that it contains water and/or an alcohol having from 1 to 4 carbon atoms, preferably ethanol or isopropanol.

**Revendications**

1. Produit pour augmenter la malléabilité et le brillant des cheveux, contenant :

    (A) un homo- ou un copolymère, formé à partir d'au moins un monomère à insaturation éthylénique de formule générale (I)

$$CH_2=CR^1R^2 \tag{I}$$

    dans laquelle $R^1$ est choisi parmi $A-(CH_2CH_2O)_x-R^3$ et COOH, A est choisi parmi $C(=O)O$, $C(=O)NH$ et $CH_2O$, x est un nombre de 1 à 100, $R^3$ est un radical alkyle en C1-C30, $R^2$ est choisi parmi H, les groupes alkyle en C1-C30 et $CH_2-R^1$, à la condition qu'au moins l'un des radicaux $R^1$ et $R^2$ contiennent le groupe $A-(CH_2CH_2O)_x-R^3$,

    (B) un polyéthylèneglycol, et

    (C) un tensioactif non-ionique ayant un indice HLB inférieur à 20.

2. Produit selon la revendication 1, caractérisé en ce que le composant (A) est un copolymère formé à partir d'au moins un monomère à insaturation éthylénique de formule générale (I), et d'au moins un monomère à insaturation éthylénique de formule générale (II)

$$CH_2=C(R^4)COOR^5 \hspace{4cm} (II)$$

dans laquelle $R^4$ et $R^5$ sont choisis indépendamment l'un de l'autre parmi H et les groupes alkyle en C1-C30.

3. Produit selon l'une des revendications précédentes, caractérisé en ce que A est choisi parmi C(=O)O et $CH_2O$, $R^2$ est choisi parmi H et le groupe méthyle, et $R^3$ est un radical alkyle en C8-C30.

4. Produit selon la revendication 1 ou 2, caractérisé en ce que, pour ce qui est du monomère de formule (I), il s'agit d'un dérivé de l'acide itaconique.

5. Produit selon l'une des revendications précédentes, caractérisé en ce que le monomère de formule (II) est l'acide acrylique, l'acide méthacrylique ou un de leurs esters alkyliques en C1-C4.

6. Produit selon l'une des revendications précédentes, caractérisé en ce que le composant (A) est choisi parmi les copolymères acide acrylique ou méthacrylique/ester polyéthoxyalkylique de l'acide acrylique ou méthacrylique, les copolymères acide acrylique ou méthacrylique/polyéthoxyalkylallyléther et les copolymères acide acrylique ou méthacrylique/ester polyéthoxyalkylique de l'acide itaconique.

7. Produit selon l'une des revendications précédentes, caractérisé en ce que les groupes acide carboxylique libre du composant (A) sont présents sous forme neutralisée.

8. Produit selon l'une des revendications précédentes, caractérisé en ce qu'il contient le composant (A) en une quantité de 0,01 à 10 % en poids.

9. Produit selon l'une des revendications précédentes, caractérisé en ce qu'il contient le polyéthylèneglycol en une quantité de 0,01 à 50 % en poids.

10. Produit selon l'une des revendications précédentes, caractérisé en ce qu'il contient le tensioactif non-ionique en une quantité de 0,01 à 20 % en poids.

11. Produit selon l'une des revendications précédentes, caractérisé en ce que le polyéthylèneglycol a une masse moléculaire supérieure à 500, de préférence de 20 000 à 3 600 000.

12. Produit selon l'une des revendications précédentes, caractérisé en ce qu'il contient en tant que tensioactif non-ionique un produit d'éthoxylation d'un alcool gras, un produit d'éthoxylation d'une amine grasse, un produit d'éthoxylation d'un alcanolamide d'acide gras ou un produit d'éthoxylation d'un ester d'acide gras.

13. Produit selon l'une des revendications précédentes, caractérisé en ce qu'il contient en outre un polymère fixatif pour cheveux, naturel ou synthétique, anionique, cationique, amphotère ou non-ionique, en une quantité de 0,01 à 30 % en poids.

14. Produit selon l'une des revendications précédentes, caractérisé en ce qu'il contient en outre un composé silicone soluble dans l'eau ou insoluble dans l'eau, en une quantité de 0,01 à 30 % en poids.

15. Produit selon l'une des revendications précédentes, caractérisé en ce qu'il contient de l'eau et/ou un alcool ayant de 1 à 4 atomes de carbone, de préférence l'éthanol ou l'isopropanol.